# FASCICULE DE BREVET EUROPEEN

(11) **EP 3 990 024 B1**
(45) Date de publication et mention de la délivrance du brevet: **21.06.2023**
(21) Numéro de dépôt: 20749927.8
(22) Date de dépôt: 25.06.2020
(51) Int. Cl.: A61K 47/32, A61K 47/34, A01K 13/00, A61K 36/23

(54) **DISPOSITIF SOUS FORME DE MATRICE POLYMERE A BASE DE CANNABINOÏDES**
VORRICHTUNG IN FORM EINER CANNABINOIDBASIERTEN POLYMERMATRIX
DEVICE IN THE FORM OF A CANNABINOID-BASED POLYMER MATRIX

(30) Priorité: 25.06.2019 US 201962866253 P; 28.04.2020 US 202063016374 P
(43) Date de publication de la demande: 04.05.2022
(73) Titulaire: AB7 Santé, 31450 Deyme (FR)
(72) Inventeur: VILBERT, Arnaud, 31450 BAZIEGE (FR); LECLERC, Sophie, 31750 ESCALQUENS (FR)
(86) Numéro de dépôt international: PCT/FR2020/000194
(87) Numéro de publication internationale: WO 2020/260777

(56) Documents cités:
- FR-A1- 3 039 991
- US-A1- 2018 318 237

## Description

La présente invention est située dans le domaine des traitements pour le soulagement des différents symptômes liés à la douleur ou au stress. Plus précisément, la présente invention concerne un dispositif d'application topique sous forme de matrice polymère renfermant au moins un actif de la famille des cannabinoïdes, seul ou en mélange avec d'autres actifs. La présente invention concerne préférentiellement le dispositif sous forme d'un collier, son utilisation pour améliorer le bien-être et la mobilité d'un animal sur lequel est appliqué le dispositif, et son procédé de préparation.

L'utilisation du cannabis dans la médecine est connue depuis l'antiquité. Les nombreux effets thérapeutiques connus à ce jour du cannabis sont dus aux molécules psychoactives présentes dans le cannabis, les cannabinoïdes, et notamment le cannabinol (CBN), le delta-9-tétrahydrocannabinol (THC) ou le cannabidiol (CBD). Le THC est majoritairement responsable des propriétés psychotropes, euphorisantes et confère le statut de stupéfiant au cannabis. Ce n'est que très récemment que l'administration de cannabidiol (CBD) un des composés actifs majeur du cannabis autrement appelé chanvre est commercialisé. Ainsi, les variétés de chanvre dépourvues de propriétés stupéfiantes (sans THC) peuvent être utilisées à des fins industrielles et commerciales sous trois conditions cumulatives :
- les variétés de chanvre autorisées figurent sur une liste inscrite dans le Code de la santé publique,
- seules les graines et les fibres peuvent être utilisées. L'utilisation des fleurs est quant à elle interdite,
- la plante doit avoir une teneur inférieure à 0.2% en delta-9-tétrahydrocannabinol (THC),
- les produits à base de CBD sont interdits s'ils contiennent du THC quelle que soit la quantité.

Ces cannabinoïdes dépourvues de THC sont utilisés pour l'amélioration de la qualité de vie qu'ils apportent aux hommes et animaux concernés par des situations telles que la douleur, le stress, l'anxiété, l'épilepsie et les spasmes, l'arthrite, les nausées ou encore les problèmes dermatologiques depuis l'antiquité. Ces cannabinoïdes étaient essentiellement administrés par la voie orale, ou sublinguale. Pour une administration par la voie topique, les huiles de cannabidiols sont formulées soit en semi-solide (pommades, crèmes, gels) soit en liquide (laits, émulsions, lotions). Les galéniques d'application topique susmentionnées nécessitent des applications répétées pour produire les effets systémiques escomptés. Par ailleurs, dans le domaine vétérinaire, l'application de tels produits topiques n'est pas aisée et non optimum pour obtenir une pénétration efficace à travers le poil de l'animal, ou pour un traitement longue durée.

Le document US2018/318237 A1 divulgue des formulations pour application dans le cou sous forme de crème comprenant le cannabidiol (CBD) présent sous forme d'huile de cannabidiol.

Un des objectifs de l'invention est de proposer un mode d'administration topique plus avantageux pour un traitement localisé de la douleur ou du stress en limitant les effets systémiques secondaires, connus de la voie orale et utilisable pour un traitement de plus longue durée. Dans le domaine qui intéresse l'invention, lorsque l'on a recours à l'application d'une composition par voie topique, on observe qu'une partie des constituants peut être amenée à disparaître, du fait de leur volatilité, dégradation par oxydation, exposition UV ou par lavage. Il en résulte un passage de tout au plus 40% de la dose efficace disponible en ingrédients actifs. De fait, la biodisponibilité des ingrédients actifs n'est pas optimale et, en conséquence son efficacité dans le soulagement de la douleur ou du stress en est réduite.

Afin d'éviter les différents inconvénients cités ci-dessus, et d'améliorer le passage des actifs à travers la peau, la demanderesse propose l'usage d'une matrice solide en polymère, en tant que véhicule d'application topique et/ou transdermique de cannabinoïdes, seuls ou en combinaison avec d'autre actifs, ladite matrice permettant d'obtenir une composition présentant des actifs incorporés stables. Par stable selon l'invention, on entend une stabilité physique du dispositif et une stabilité chimique des actifs au sein du dispositif. Par stabilité physique, on entend l'absence d'exsudation confirmant la bonne incorporation de la phase active, la tenue mécanique du dispositif, notamment la résistance à la déformation, la souplesse ou dureté requise pour l'utilisation finale. Par stabilité chimique, on entend l'absence de dégradation de l'actif principal ou des actifs, ladite dégradation de l'actif devant être inférieure à 10% de la quantité initialement introduite dans le dispositif. Le dispositif selon l'invention doit être stable à l'évaporation, risque de perte notamment par lessivage, et proposant, à l'utilisation, une diffusion continue, contrôlée, efficace et progressive des actifs sur le sujet à traiter.

Un des objectifs de la présente invention est également de proposer un dispositif dont le procédé de fabrication reste simple à mettre en oeuvre et transposable à l'échelle industrielle. L'art antérieur dans le domaine des polymères décrit des difficultés d'incorporation d'actifs au sein desdits polymères. Il est en effet nécessaire de pouvoir incorporer de façon totale et stable l'actif au sein du polymère, en évitant la libération ou relargage non contrôlé. En réponse à ces problèmes l'art antérieur décrit des solutions diverses telles que l'ajout de nombreux ingrédients spécifiques pour rendre incorporable l'actif, ou propose d'intégrer l'actif au sein de structures de type particulaire plus ou moins complexes à réaliser. Le procédé d'obtention du dispositif selon l'invention permet d'obtenir l'incorporation des actifs liquides, au sein du polymère de structure de la matrice sans avoir recours à l'utilisation d'ingrédients spécifiques ou d'intégrer l'actif sous une forme particulaire quelconque.

Le dispositif selon la présente invention peut être définie d'application topique, car il est à usage local, appliqué sur la peau ou le pelage du sujet à traiter. Le dispositif de la présente invention peut également répondre à la définition de l'homme de l'art d'un dispositif transdermique puisque constitué d'une matrice polymère servant de support aux actifs et réalisé afin de permettre la libération desdits actifs pour leur diffusion chez le sujet à traiter afin que les actifs puissent atteindre les récepteurs aux cannabinoïdes. Dans la suite de la demande, on considérera indifféremment les termes « topique » et « transdermique » appliqués au dispositif de la présente invention.

Les systèmes transdermiques contenant des dérivés de cannabinoïdes sont décrits dans l'art antérieur. Cependant, ceux-ci décrivent tous des systèmes de type réservoir ou matriciel, constitués d'au moins deux, voire plusieurs couches de matériaux différents ayant des fonctions diverses. Ces systèmes transdermiques sont constitués d'un moins une couche adhésive afin de fixer le dispositif sur le sujet, d'une couche réservoir contenant le ou les actifs à véhiculer et d'une couche externe imperméable. L'art antérieur ne décrit pas de système non-adhésif et monocouche permettant d'incorporer simplement et de diffuser un actif de type cannabinoïde tel que le propose la présente invention. De plus les systèmes décrits sont complexes à réaliser et non adaptés à l'usage sur un sujet animal recouvert de pelage.

Dans la présente invention, la demanderesse propose donc l'utilisation d'au moins du cannabidiol (CBD) exempt de delta-9-tétrahydrocannabinol (THC), seul ou en association avec d'autres actifs en tant qu'ingrédients actifs antidouleur ou anti-stress au sein d'une matrice polymère, monocouche, et monopolymèrique jamais décrite dans l'art antérieur. Par matrice polymère monocouche selon la présente invention, on entend une matrice constituée d'une seule couche de polymère et non la juxtaposition de plusieurs couches telle que couramment décrit dans l'art antérieur. Par monopolymérique selon l'invention, on entend une matrice constituée d'un même polymère. Ledit même polymère peut quant à lui être un homopolymère ou un copolymère. Un homopolymère est un polymère issu d'une seule espèce (réelle, implicite ou hypothétique) de monomère. Tous les motifs de répétition d'un homopolymère sont de même nature chimique. Un copolymère est un polymère issu d'au moins deux monomères différents, appelés comonomères; il contient au moins deux types de motifs de répétition..

### Brève description des figures

Figure 1 illustre la cinétique de libération du CBD à partir du dispositif selon une réalisation de l'invention selon l'exemple 10.
Figure 2 illustre la cinétique de libération du CBD à partir des dispositifs selon des réalisations de l'invention selon les exemples 5 et 11.

De manière surprenante, il a été constaté qu'en incorporant le cannabidiol (CBD) exempt de delta-9-tétrahydrocannabinol (THC), sous forme d'isolat ou d'huile riche en cannabinoïdes, dérivés de chanvre, en tant que seul actif ou préférentiellement en combinaison avec des huiles essentielles dans une matrice solide en polymère telle que décrite dans la présente invention, il en résulte une composition stable et un soulagement efficace de la douleur ou du stress qui se manifeste chez le sujet humain ou animal traité au moyen du dispositif conforme à l'invention. Il existe donc un besoin de la présente invention qui concoure au soulagement de la douleur ou du stress, et permet d'augmenter la stabilité de la composition tout en garantissant la biodisponibilité desdits constituants pour l'obtention d'un effet de soulagement de douleurs ou de stress plus efficace, et d'une durée d'efficacité allongée.

Par conséquent, un premier objet de la présente invention consiste en un dispositif comprenant une matrice solide en polymère, incorporant une composition d'actifs contenant, au moins un premier ingrédient actif constitué par du cannabidiol (CBD) exempt de delta-9-tétrahydrocannabinol (THC) sous forme d'huile ou d'isolat extrait du chanvre, ou cannabis, seul ou en association avec d'autres actifs. Les actifs utilisés en association avec le cannabidiol (CBD) exempt de delta-9-tétrahydrocannabinol (THC), selon la présente invention, peuvent être choisis parmi les anti-douleurs, les anti-inflammatoires, les actifs apaisants ou anesthésiants. Selon un autre mode de l'invention le cannabidiol (CBD) exempt de delta-9-tétrahydrocannabinol (THC) peut être également associé à des actifs insecticides ou répulsifs contre les nuisibles.

Selon la présente invention, le cannabidiol (CBD) exempt de delta-9-tétrahydrocannabinol (THC) est préférentiellement utilisable sous forme de poudre, liquide, solution, huile ou isolat, extrait de la plante de chanvre. Par huile de CBD, on entend préférentiellement une huile riche en cannabinoïdes, on entend plus particulièrement riche en phytocannabinoïdes et terpènes identifiés dans les gênes du chanvre. Lesdites huiles sont notamment les huiles issues des plantes Cannabis sativa, Cannabis indica et Cannabis ruderalis. De préférence, l'huile de CBD utilisée contient entre 50 et 95% de cannabidiol (CBD) et préférentiellement entre 80 et 90% de CBD en addition à différents terpènes. L'huile utilisée est une huile, appelée huile à spectre large (« Broad spectrum »), exempte de THC et concoure cependant au soulagement des douleurs ou du stress ou tout autre pathologie y étant relative. Dans la suite du texte on utilisera indifféremment, « huile de CBD » ou « huile de chanvre riche en CBD » pour désigner le CBD sous forme d'une huile extraite du chanvre riche en Cannabidiol (CBD), et qui est exempte de THC. Dans la composition selon l'invention, l'huile de CBD est présente en quantité comprise entre 0.01 et 10 % p/p, préférentiellement entre 0.1 et 5% en poids total de dispositif. Le système de purification et d'extraction des cannabidiol et terpènes peut notamment mais non exclusivement être basé sur une technologie brevetée qui préserve les terpènes et les lipides de la plante, en s'affranchissant des composés non désirés comme le Delta-9-THC, des cires et la chlorophylle, tel que décrit notamment dans la demande de brevet US2018/0333446.

De façon particulièrement préférée, le CBD utilisé est sous forme d'un isolat. Par isolat selon la présente invention, on entend notamment une poudre cristalline obtenue par extraction de la plante de chanvre, avec une concentration en CBD supérieure ou égale à 99%. Dans un mode préférentiel, l'isolat de CBD utilisé dans la composition est présent en quantité entre 0.01 et 10% p/p, préférentiellement entre 0.1% et 5% p/p, plus préférentiellement entre 0.5% et 2.5% p/p de la composition.

Dans un mode préférentiel selon la présente invention, les actifs utilisables en combinaison avec le cannabidiol exempt de THC sont des huiles essentielles, ou des dérivés monoterpéniques des huiles essentielles de type alcools, aldéhydes ou esters. Préférentiellement, ces actifs sont choisis parmi le linalol, le menthol ou les huiles essentielles de menthe poivrée, lavande, lavandin, helicryse italienne, cèdre, citron, citronnelle, carotte, gingembre, niaouli, orange douce, clou de girofle, Eucalyptus citriodora, Eucalyptus radiata, géranium, valériane, Nepeta Cataria, camomille, Ylan Ylang, sauge sclarée, mandarine, bois de santal, bergamote, marjolaine, encens, géranium, thym, genévrier ou leurs mélanges. D'une manière préférentielle l'huile de CBD ou l'isolat de CDB sera associée au menthol, au Linalol, à l'huile essentielle de menthe poivrée, d'Eucayptus citriodora, de lavande, lavandin, valériane, ou Nepeta Cataria. Plus préférentiellement, dans le dispositif apaisant selon l'invention, les actifs sont l'isolat de CBD ou l'huile de CBD et l'huile essentielle de menthe poivrée ou l'huile d'Eucayptus citriodora pour leur action apaisante et/ou anti-douleur couplée à celle du CBD. Dans le dispositif selon l'invention, les actifs sous forme d'huiles essentielles ou composés des huiles essentielles sont présents en quantité comprise entre 0.5 et 20% p/p, préférentiellement entre 0.5 et 15% p/p, et plus préférentiellement entre 0.5 et 6% en poids total du dispositif.

Selon une première variante de réalisation, la matrice polymère est constituée de polymères thermodurcissables ou thermodurs, choisis parmi le polyuréthane coulé, les silicones réactives et les résines époxy, préférentiellement le polyuréthane coulé. La matrice en polyuréthane coulé, conforme à la présente invention, est décrite dans le brevet FR2992325.

Selon une seconde variante préférée de réalisation du dispositif selon l'invention, les polymères constituant la matrice solide sont choisis parmi les polymères thermoplastiques biodégradables ou non, choisis dans le groupe formé par les polyoléfines et leurs dérivés, les éthylènebutyl acrylates, les polyamides, les copolyamides et leurs dérivés, les polychlorures de vinyles (PVC), les polyuréthanes (TPU) et leurs dérivés, les styréniques et leurs dérivés, les thermoplastiques vulcanisés, les agropolymères et leurs dérivés, les polyesters et leurs dérivés, pris seul ou en mélange. Les polyoléfines et dérivés peuvent être notamment choisies parmi les polyéthylènes (PE), les polypropylènes (PP), les copolymères d'éthylène et d'acétate de vinyle (EVA). Les styréniques et leurs dérivés peuvent être choisis notamment parmi les copolymères polystyrène-poly(éthylène-butylène)-polystyrènes (SEBS), les copolymères polystyrène-polyisoprène-polystyrène (SIS), les copolymères polystyrène- polybutadiène-polystyrènes (SBS). Les copolyamides et leurs dérivés peuvent être notamment choisis parmi les éthers bloc amides (EBA). Les agropolymères et leurs dérivés peuvent notamment être choisis parmi les polysaccharides, l'amidon, la cellulose et dérivés, les protéines.

Préférentiellement le polymère thermoplastique utilisable selon l'invention est choisi parmi une polyoléfine et leurs dérivés ou un polyuréthane et leurs dérivés. De manière plus préférentielle, la matrice polymère constituant le dispositif selon la présente invention est un thermoplastique constitué de copolymères d'éthylène et d'acétate de vinyle (EVA) ou de polyuréthane à motif polyéther ou polyester (TPU). En effet, la demanderesse a démontré que ces deux polymères préférés conféraient des propriétés de chargement et de diffusion des actifs optimales. Ces deux polymères permettent de conférer à la fois les propriétés mécaniques d'élasticité ou de dureté recherchées pour l'usage du dispositif et, des capacités d'incorporations et de libération optimales des actifs selon un procédé facilement mis en oeuvre à l'échelle industrielle. Préférentiellement, pour un dispositif à l'aspect transparent, translucide, le TPU sera préféré. Le polymère représente entre 40 et 99% en poids du dispositif. Préférentiellement la matrice polymère est en quantité suffisante pour compléter les 100% (qsp 100%) de la composition du dispositif.

Selon un mode particulièrement préféré, lorsque le dispositif est un collier pour animal, le polymère thermoplastique utilisable selon l'invention est non-adhésif. En effet, un dispositif adhésif ne serait pas adapté à une application sur le pelage de l'animal.

Selon un autre mode particulièrement préféré, pour répondre à l'objectif de maintien de la stabilité et de l'intégrité du dispositif pour un traitement du sujet sur du long terme, le polymère thermoplastique utilisable selon l'invention n'est pas hydrosoluble.

Par ailleurs, à la différence des matrices polymères rencontrées dans l'art antérieur constituées d'un mélange de polymère et/ou de plusieurs couches ou séquences de polymères, il est précisé que la matrice constituant le dispositif selon l'invention est une matrice uniquement monocouche et monopolymérique, et non constituée par une association de plusieurs séquences de polymères différents .

Dans la présente invention, l'homme de l'art pourra ajouter d'autres additifs à la formulation du dispositif. A titre d'exemples non exhaustifs, les additifs de formulation peuvent être des dispersants, solvants, vectorisants, propénétrants, plastifiant, stabilisant, colorants, ou parfums, afin d'être ajoutés à la composition selon l'invention en fonction de l'effet recherché.

A titre d'exemple de dispersants, solvants, vectorisants, ou propénétrants, on peut nommer, les huiles d'origine organique, synthétique, minérale ou végétale, les esters tels que les mélanges de succinate, glutarate et adipate, les huiles de type acide gras polyinsaturé oméga 3, les esters d'acide gras, les éthers de glycol, ou le squalène et ses dérivés.

Dans un mode préférentiel le dispositif selon l'invention comprend des vectorisants, ou propénétrants pouvant faciliter la vectorisation de l'actif sur la peau ou le pelage du sujet et/ou le passage transcutané des actifs. Dans un mode préféré ces vectorisants, propénétrants sont des composés lipophiles. On peut citer à titre indicatif l'huile de coprah, l'huile de jojoba, l'huile de chanvre, l'huile de macadamia, l'huile de lin ou l'huile d'amande douce, l'huile de krill, le diethylene glycol monoethyl ether, commercialisé sous le nom de Transcutol V, le myristate d'isopropyle, ou des dérivés du squalène, préférentiellement le 2,6,10,15,19,23-hexamethyltetracosane, appelé également perhydrosqualene ou dodecahydrosqualene commercialisé sous le nom de Squalane. Ces ingrédients seront présents en quantité comprise entre 0.1 et 30% p/p, préférentiellement entre 0.5 et 15% p/p, plus préférentiellement entre 1 et 6% en poids total du dispositif.

Selon la présente invention, le dispositif comprend également un composé plastifiant. En effet, la demanderesse a constaté de façon surprenante que le plastifiant avait un rôle dans la libération des actifs de la matrice polymère, et que la nature et la concentration de plastifiant permettait de moduler la libération des actifs. A titre de plastifiant utilisable au sein du dispositif selon l'invention, on peut citer le phosphate d'Ethylhexyldiphenyl, l'adipate de dioctyle, l'adipate de diisooctyle, l'adipate de dibutyle, l'adipate de diméthyle, le glutarate de diméthyle, le succinate de diméthyle, pris seul ou en mélange. Selon un mode particulier le mélange de plastifiant composé d'adipate de diméthyle, de glutarate de diméthyle, et de succinate de diméthyle est utilisé sous la forme du mélange commercial appelé Coasol. De manière préférentielle, les plastifiants seront différents des phtalates, dont l'utilisation est controversée due à leur toxicité. Dans un mode préféré selon l'invention, le plastifiant ou le mélange de plastifiant est présent en quantité comprise entre 0 et 50% en poids, préférentiellement entre 5 et 35% en poids total de la composition. Additionnellement, le plastifiant permet de conférer à la matrice les propriétés plastiques recherchées pour son utilisation. Selon la présente invention, le plastifiant a également un rôle d'assouplissant des matériaux polymères utilisés afin de conférer au dispositif la souplesse ou la dureté choisie. En effet, à titre d'exemple préféré, un des objets de la présente invention est un dispositif sous la forme d'un collier pour animal. Ledit collier nécessite donc une souplesse suffisante pour être adapté à la forme du cou de l'animal mais tout en conservant la forme, la structure voulue et les propriétés de diffusion de l'actif recherchées. Selon un mode particulièrement préféré selon l'invention, notamment lorsque le polymère utilisé est le TPU, la matrice polymère selon l'invention comprend au moins un plastifiant.

Dans un mode particulier, des stabilisants et/ou antioxydants pourront être ajoutés à la composition du dispositif selon l'invention à une concentration comprise entre 0 et 5% p/p, préférentiellement entre 0 et 1.5% en poids total de la composition. A titre d'exemple préférentiel, on peut citer la vitamine E ou tocophérol, qui sera présent entre 0.5 et 1.5% en poids total de la composition.

Des colorants ainsi que des parfums pourront également être ajoutés à la composition. Les colorants peuvent être présents sous forme liquide ou solide. A titre d'exemple de parfum, le parfum est choisi parmi le parfum de vanille, de citron vert, de lavande, de violette, de pomme, d'abricot, de patchouli, de bambou feuilles vertes et peut être adapté à l'indication et à l'utilisateur.

L'homme de l'art veillera à choisir les additifs de formulation afin qu'ils n'interfèrent pas sur les propriétés de la composition recherchées selon l'invention.

Selon une variante de réalisation, le dispositif selon l'invention comprend, en % en poids du dispositif :
- Entre 0.01 et 10 % d'isolat de CBD exempt de THC ou d'huile de CBD exempte de THC,
- Entre 0.5 et 20% d'actifs complémentaires,
- Entre 0.1 et 30% d'additifs vectorisants,
- Entre 0 et 50% d'additifs plastifiants,
- Qsp 100% en poids de matrice solide en polymère, choisi parmi l'EVA ou le TPU.

Selon une variante de réalisation particulière, le dispositif selon l'invention comprend, en % en poids du dispositif :
- Entre 0.01 et 10 % d'isolat de CBD exempt de THC ou d'huile de CBD exempte de THC,
- Entre 0.5 et 20% d'actifs complémentaires,
- Entre 0.5 et 15% d'additifs vectorisants,
- Entre 0 et 1.5% d'additifs de type anti-oxydant,
- Qsp 100% en poids de matrice solide en polymère d'EVA.

Selon un autre mode particulier de réalisation, le dispositif selon l'invention comprend, en % en poids du dispositif :
- Entre 0.1 et 5 % d'isolat de CBD exempt de THC ou d'huile de CBD exempte de THC,
- Entre 0.5 et 15% en poids d'actifs sous forme d'huile essentielle et/ou de composés d'huiles essentielles,
- Entre 0.5 et 15% d'additifs vectorisants,
- Entre 5 et 35% d'additifs plastifiants,
- Qsp 100% en poids de matrice solide en polymère TPU.

Selon un mode plus préférentiel de réalisation, le dispositif selon l'invention comprend, en % en poids du dispositif :
- Entre 0.1 et 5 % d'isolat de CBD exempt de THC,
- Entre 0.5 à 15% en poids d'actifs sous forme d'huile essentielle et/ou de composés d'huiles essentielles,
- Entre 1 et 6 % d'additifs de type vectorisants,
- Entre 5 et 35% d'additifs de type plastifiants,
- Qsp 100% en poids de matrice solide en polymère, TPU.

Selon un second mode plus préférentiel de réalisation, le dispositif selon l'invention comprend, en % en poids du dispositif :
- Entre 0.1 et 5 % d'isolat de CBD exempt de THC,
- Entre 0.5 à 15% en poids d'actifs sous forme d'huile essentielle et/ou de composés d'huiles essentielles, plus préférentiellement l'huile essentielle de menthe poivrée,
- Entre 1 et 6 % d'additifs de type vectorisants, plus préférentiellement le squalane,
- Entre 5 et 35 % de plastifiants, préférentiellement un mélange de phosphate d'Ethylhexyldiphenyl, d'adipate de diméthyle, de glutarate de diméthyle et de succinate de diméthyle ou de glutarate de diméthyle.
- Qsp 100% en poids de matrice solide en polymère, composé de TPU.

Selon un mode de réalisation, le dispositif se présente sous la forme d'un collier, d'un bracelet, d'un médaillon, d'un patch monocouche ou d'une enduction textile. De façon préférentielle, le dispositif est réalisé sous forme d'un collier ou d'un bracelet. Dans un mode préféré le procédé d'obtention du dispositif est un procédé de moulage par injection. Dans un mode particulièrement préféré, le dispositif est un collier pour animal de compagnie et notamment un collier pour chien ou chat.

La présente invention a pour second objet le dispositif consistant en une matrice polymère solide incorporant une solution d'actifs contenant au moins un isolat de CBD exempt de THC ou une huile de CBD exempte de THC pour son utilisation dans le traitement ou le soulagement de douleurs ou du stress chez un sujet humain ou animal. Plus particulièrement, eu égard aux propriétés du CBD en combinaison avec le ou les autres actifs selon l'invention, le dispositif permet par son application de réguler les douleurs induites, notamment par des désordres inflammatoires, immuno-induits ou non. A titre d'exemple, le dispositif permet de réguler les douleurs cutanées ou le prurit, les douleurs musculaires, les douleurs articulaires, les tendinites ainsi que les douleurs rhumatismales comme par exemple l'arthrose ou l'arthrite chez un sujet humain et animal. Le dispositif selon l'invention peut être également utilisé pour réguler le stress d'un sujet humain ou animal.

La présente invention concerne donc le dispositif d'application topique/transdermique, en matrice polymère thermoplastique chargée de cannabidiol (CBD) exempt de delta-9-tétrahydrocannabinol (THC), pour son utilisation dans le soulagement de douleurs ou du stress chez un sujet humain ou animal à traiter. Ledit dispositif est caractérisé en ce qu'il est mis en contact avec la peau et/ou le pelage. Selon un mode préférentiel, ledit dispositif permet d'assurer le soulagement des douleurs ou du stress d'un animal, caractérisé en ce qu'il est appliqué au contact de la peau et/ou le pelage sous la forme d'un collier. Les actifs sont ainsi relargués du collier et, par application topique, pénètrent dans la peau, notamment transportés via le sébum. La présente invention concerne donc également le dispositif pour son utilisation pour améliorer la mobilité et le bien-être d'un sujet et plus particulièrement d'un animal.

La présente invention a également pour objet le dispositif selon l'invention pour son utilisation pour administrer le cannabidiol, et tout autre actif incorporé au sein du dispositif, par la voie transdermique. Cette voie offre ainsi l'avantage de diminuer tout effet métabolique classiquement obtenu lors de l'administration de l'actif par voie orale et d'augmenter la biodisponibilité des composés actifs. De plus, l'utilisation du dispositif transdermique selon l'invention présente l'avantage de délivrer l'actif de façon progressive, continue et mesurée, et sur une plus longue période de temps. Le temps d'application souhaité des dispositifs selon l'invention est compris entre 30 min pour un effet rapide à plusieurs mois pour un effet à long terme. Selon un mode préféré, le dispositif selon l'invention est appliqué pendant une durée de 1 à 60 jours, préférentiellement pendant 28 jours (4 semaines). En effet selon un mode préféré ce temps de 4 semaines est un temps nécessaire et suffisant pour traiter un sujet humain ou animal contre les douleurs ou le stress avant une possible nouvelle application si nécessaire.

La présente invention a également pour objet procédé de réalisation du dispositif selon l'invention. En effet, de façon surprenante, le choix du procédé et notamment des températures de mélange, permet de réaliser un procédé en peu d'étapes et sans avoir recours à l'addition d'ingrédients spécifique ou de structure particulaires pour aider à l'incorporation des actifs au sein de la matrice polymère.

Selon la présente invention, le procédé de préparation du dispositif selon l'invention comprend les 4 étapes principales suivantes : la mise en température du polymère, la préparation de la phase active, le mélange de la phase active et du polymère, la mise en forme du dispositif.

La première étape du procédé consiste donc à introduire le polymère dans un réacteur préalablement chauffé à la température requise en fonction de la nature du polymère. Dans un mode préféré, le polymère sera chauffé à une température comprise entre 10 à 15 degrés Celsius en dessous de sa température de fusion, à une température suffisante pour conférer une structure au polymère permettant d'ouvrir les réseaux pour faciliter l'incorporation des actifs sans atteindre la fusion dudit polymère.

A titre d'exemple dans un mode particulier selon l'invention quand les polymères utilisés sont les polyuréthanes (TPU), le réacteur sera chauffé entre 80 et 90 degrés Celsius, préférentiellement entre 90 et 95°C. Dans un autre mode particulier selon l'invention, quand les polymères utilisés sont les copolymères d'éthylène et d'acétate de vinyle (EVA), le réacteur sera chauffé entre 45 et 90 degrés Celsius, préférentiellement entre 70 et 75°C. Les réacteurs sont ensuite mis sous agitation.

La seconde étape du procédé consiste à préparer la phase active liquide selon le protocole suivant : peser et mélanger les ingrédients de type plastifiants et vectorisants, et mettre sous agitation pour homogénéiser afin d'obtenir un mélange limpide. Les actifs sont ensuite ajoutés sous agitation à température ambiante jusqu'à obtention d'un mélange limpide, translucide homogène.

La troisième étape du procédé consiste en l'addition progressive de la phase active au polymère lorsque la température cible choisie d'incorporation est atteinte. L'agitation est maintenue jusqu'à ce que les actifs soient totalement incorporés dans le polymère. L'ensemble est refroidi à température ambiante en maintenant l'agitation. Les colorants ou autres additifs sont ajoutés dans le réacteur. Le réacteur est ensuite vidangé et on procède ensuite à la quatrième et dernière étape d'injection-moulage afin d'obtenir la forme souhaitée du dispositif selon les procédés d'injection-moulage connus de l'homme de l'art.

La présente invention a également pour objet le produit obtenu par le procédé défini précédemment.

En résumé, l'invention concerne un dispositif d'application topique constitué d'une matrice monocouche en polymère caractérisé en ce que le polymère est choisi parmi le groupe consistant en un copolymère d'éthylène et d'acétate de vinyle et un polyuréthane, ladite matrice étant également caractérisée en ce qu'elle comprend au moins du cannabidiol (CBD) exempt de delta-9-tétrahydrocannabinol (THC) et entre 0.5% et 20% en poids total de la matrice d'au moins une huile essentielle ou un dérivé monoterpénique des huiles essentielles.

L'invention concerne plus particulièrement un dispositif, caractérisé en ce que le polymère est non-adhésif.

. L'invention concerne plus particulièrement un dispositif, caractérisé en ce que le polymère est un copolymère d'éthylène et d'acétate de vinyle ou un polyuréthane.

L'invention concerne ledit dispositif, caractérisé ce que le cannabidiol est exempt de delta-9-tétrahydrocannabinol. De façon plus préférentielle, ledit dispositif est caractérisé en ce que la cannabidiol est présent sous forme d'un isolat de CBD ou d'une huile de CBD, ledit isolat de CBD contient au moins 90% de CBD et ladite huile de CBD comprend entre 80 et 90% de CBD.

L'invention concerne également le dispositif précédent caractérisé en ce qu'il comprend également au moins une huile essentielle ou des dérivés monoterpéniques des huiles essentielle, choisis parmi le linalol, le menthol ou les huiles essentielles de lavande, lavandin, helicryse italienne, cèdre, citron, citronnelle, carotte, gingembre, niaouli, orange douce, clou de girofle, Eucalyptus citriodora, Eucalyptus radiata, menthe poivrée, géranium, valériane, Nepeta Cataria, camomille, Ylan Ylang, sauge sclarée, mandarine, bois de santal, bergamote, marjolaine, encens, géranium, thym, genévrier ou leurs mélanges.

Le dispositif selon la présente invention est également caractérisé en ce qu'il comprend un composé vectorisant choisi parmi les huiles d'origine organique, synthétique, minérale ou végétale, les esters tels que les mélanges de succinate, glutarate et adipate, les huiles de type acide gras polyinsaturé oméga 3, les esters d'acide gras, les éthers de glycol, ou le squalène et ses dérivés.

Dans le cas de polymère particulier, et notamment dans le cas où le polymère utilisé est le TPU, le dispositif est caractérisé en ce qu'il comprend également un composé plastifiant choisi parmi le phosphate d'ethylhexyldiphenyl, l'adipate de dioctyle, l'adipate de diisooctyle, l'adipate de dibutyle, l'adipate de diméthyle, le glutarate de diméthyle, le succinate de diméthyle, pris seul ou en mélange.

Selon la présente invention, le dispositif est également caractérisé en ce qu'il se présente sous la forme d'un collier, d'un bracelet, d'un patch, d'un médaillon, ou d'une enduction textile, et préférentiellement sous la forme d'un collier.

Plus particulièrement l'invention concerne un dispositif, d'application topique, en matrice polymère thermoplastique, chargée d'ingrédients actifs pour son utilisation dans le soulagement de douleurs ou du stress chez un animal, caractérisé en ce que ledit dispositif est mis en contact avec la peau et/ou le pelage, et en ce que le dispositif comprend au moins un isolat de cannabidiol, exempt de delta-9-tétrahydrocannabinol, au moins une huile essentielle ou un composé d'une huile essentielle et au moins un polymère thermoplastique non -adhésif choisi parmi un polyuréthane ou un copolymère d'éthylène et d'acétate de vinyle.

La présente invention concerne également le dispositif défini précédemment pour son utilisation dans le soulagement du stress ou des douleurs musculaires, articulaires, ou rhumatismales, telles les tendinites, arthrite et l'arthrose.

L'invention concerne également l'utilisation d'un dispositif selon la présente invention caractérisé en ce qu'il permet de moduler la libération et la diffusion du cannabidiol sur un sujet humain ou animal par la voie topique ou transdermique.

L'invention concerne également un procédé de fabrication d'un dispositif consistant en une matrice polymère thermoplastique comprenant au moins un polymère thermoplastique non-adhésif choisi parmi un polyuréthane ou un copolymère d'éthylène et d'acétate de vinyle, ladite matrice étant également caractérisée en ce qu'elle comprend au moins un isolat de cannabidiol, exempt de delta-9-tétrahydrocannabinol ou une huile de cannabidiol exempte de de delta-9-tétrahydrocannabinol et entre 0.5 et 20% p/p d'au moins une huile essentielle ou un composé d'une huile essentielle, ledit procédé comprenant les étapes de mise en température choisie du polymère, de préparation de la phase active, de mélange de la phase active avec le polymère, puis la mise en forme du dispositif, caractérisé en ce que ledit procédé ne comporte pas d'étape d'addition d'excipients spécifiques ou de structure particulaires pour aider à l'incorporation des actifs au sein de la matrice polymère.

L'invention concerne également le produit obtenu par le procédé précédemment décrit.

Les exemples ci-dessous servent à illustrer l'invention sans en restreindre sa portée. Bien que réalisés et définis sous une forme particulière (collier, bracelet, ...) les compositions des exemples suivants peuvent être adaptés à différentes formes finales. Seul le procédé d'injection-moulage final sera modifié et adapté en fonction de la forme définitive recherchée.

### Exemple 1 : Collier anti-douleur

Fabrication d'un collier en matrice en copolymère d'éthylène et d'acétate de vinyle (EVA) chargée en huile de chanvre riche en CBD

| **Composition** | **Concentration (% p/p)** |
|---|---|
| Transcutol V | 5 |
| Huile de chanvre riche en CBD | 2 |
| Huile de lin | 3 |
| Huile de menthe poivrée | 0.75 |
| EVA | QSP 100 |

Dans un bêcher, on introduit, à la température ambiante, les actifs, à savoir l'huile de chanvre riche en cannabinoïde et l'huile de menthe poivrée. On rajoute l'huile de lin, on homogénéise le mélange puis on ajoute le transcutol V en guise de propénétrant. On agite faiblement à l'aide d'un barreau aimanté afin d'obtenir un mélange homogène qui constitue la solution d'actif antidouleur.

Ensuite, on préchauffe un mélangeur cylindrique à 70°C dans un bain d'huile. On y introduit, sous faible agitation, les granulés d'EVA (ALCUDIA^{®} PA-538) jusqu'à ce que la température mesurée au sein desdits granulés avoisine 70°C. Ensuite, on introduit dans le mélangeur, toujours sous faible agitation, la solution obtenue précédemment. On laisse agiter jusqu'à ce que tout le liquide soit entièrement absorbé par le polymère puis on baisse la température à 25°C. On vidange le mélangeur, le compound, ainsi obtenu est stocké dans un emballage hermétique à l'air et à l'humidité. Par « compound » on entend le terme bien connu de l'homme de l'art pour désigner le polymère chargé d'actif.

On injecte le polymère chargé d'actifs obtenu ci-dessus en collier pesant environ 41g en sortie de buse pour une longueur de 75 cm. Ce collier est destiné à être porté par un chien autour du cou pour soulager les douleurs diverses de type arthrose.

### Exemple 2 : Collier anti-douleur

| **Composition** | **Concentration (% p/p)** |
|---|---|
| Huile de chanvre riche en CBD | 1 |
| Myritate d'isopropyle | 1.5 |
| Linalol | 3 |
| Menthol | 2.5 |
| Huile de lin | 2 |
| EVA | QSP 100 |

Dans un bêcher, on introduit, à la température ambiante, les 3 actifs, à savoir successivement l'huile de chanvre, le linalol et le menthol. On rajoute l'huile de lin raffinée afin de mieux disperser les actifs puis le myristate d'isopropyl en tant que propénétrant. On agite faiblement à l'aide d'un barreau aimanté afin d'obtenir un mélange homogène qui constitue la solution d'actifs antidouleur.

Ensuite, on préchauffe un mélangeur cylindrique à 70°C dans un bain d'huile. On y introduit, sous faible agitation, les granulés d'EVA (ALCUDIA^{®} PA-538) jusqu'à ce que la température mesurée au sein desdits granulés avoisine 70°C. Ensuite, on introduit dans le mélangeur, toujours sous faible agitation, la solution obtenue précédemment. On laisse agiter jusqu'à ce que tout le liquide soit entièrement absorbé par le polymère puis on baisse la température à 25°C. On vidange le mélangeur, le compound ainsi obtenu est stocké dans un emballage hermétique à l'air et à l'humidité.

On injecte le polymère chargé d'actifs obtenu ci-dessus en collier pesant environ 41g en sortie de buse pour une longueur de 75 cm. Ce collier est destiné à être porté par un chien autour du cou pour soulager des douleurs diverses de type arthrose.

### Exemple 3 : Médaillon anti-stress

| **Composition** | **Concentration (% p/p)** |
|---|---|
| Transcutol V | 3 |
| Huile de chanvre riche en CBD | 5 |
| Huile essentielle de valériane | 2 |
| EVA | QSP 100 |

Dans un bêcher, on introduit, à la température ambiante, les actifs, à savoir l'huile de chanvre riche en cannabinoïde et l'huile essentielle de valériane, puis on rajoute le Transcutol V en tant que propénétrant. On agite faiblement à l'aide d'un barreau aimanté afin d'obtenir un mélange homogène qui constitue la solution d'actifs relaxants, antistress.

Ensuite, on préchauffe un mélangeur cylindrique à 70°C dans un bain d'huile. On y introduit, sous faible agitation, les granulés d'EVA (ALCUDIA^{®} PA-538) jusqu'à ce que la température mesurée au sein desdits granulés avoisine 70°C. Ensuite, on introduit dans le mélangeur, toujours sous faible agitation, la solution d'actifs obtenue précédemment. On laisse agiter jusqu'à ce que tout le liquide soit entièrement absorbé par le polymère puis on baisse la température à 25°C. On vidange le mélangeur, le compound ainsi obtenu est stocké dans un emballage hermétique à l'air et à l'humidité.

On injecte le polymère chargé d'actifs obtenu ci-dessus en médaillon pesant environ 15g en sortie de buse. Ce médaillon est destiné à être porté en collier par un chien pour l'apaiser en cas de circonstances susceptibles de générer du stress.

### Exemple 4 : Bracelet anti douleur

| **Composition** | **Concentration (% p/p)** |
|---|---|
| Transcutol V | 3 |
| Huile de chanvre riche en CBD | 4 |
| Menthol | 1.5 |
| Huile de macadamia raffinée | 2 |
| Huile essentielle de lavande angustifolia | 5.5 |
| EVA | QSP 100 |

L'exemple 4 est l'élaboration d'une matrice active monopolymérique pouvant être injecté sous forme d'un bracelet. Le procédé de fabrication est similaire à celui des exemples 1 à 3 ci-dessus

### Exemple 5 : Collier apaisant

| **Composition** | **Concentration (% p/p)** |
|---|---|
| Squalane | 1 |
| Isolat de CBD | 0.75 |
| Huile essentielle de menthe poivrée | 0.75 |
| Ethylhexyldiphenyl phosphate | 15 |
| adipate de diméthyle, glutarate de diméthyle, et succinate de diméthyle | 8.5 |
| Colorant | 0.1 |
| TPU | QSP 100 |

Introduire le polymère dans un réacteur préalablement chauffé à la température requise en fonction de la nature du polymère, ici pour le TPU, autour de 90-95°C, à une température suffisante pour conférer une structure au polymère permettant d'ouvrir les réseaux pour y incorporer les actifs. Mettre sous agitation.

Préparer la phase active liquide selon le protocole suivant :
- Peser et mélanger les ingrédients de type plastifiants et vectorisants, et mettre sous agitation pour homogénéiser afin d'obtenir un mélange limpide,
- Sous agitation à température ambiante, ajouter l'isolat de CBD en tant que premier actif, et laisser agiter jusqu'à obtention d'un mélange limpide translucide
- Ajouter le co-actif, ici l'huile essentielle de menthe poivrée et homogénéiser pour obtenir un mélange translucide homogène,

Une fois que le polymère est à la température cible, ajouter progressivement le mélange d'actif préparé précédemment.

Laisser agiter jusqu'à ce que la phase active soit totalement incorporée dans le.

Refroidir à Température ambiante en maintenant l'agitation.

Ajouter le colorant et homogénéiser le mélange.

Vidanger le réacteur et procédé aux étapes d'injection-moulage afin d'obtenir la forme souhaitée du dispositif.

Les exemples selon l'invention sont testés en stabilité physique et chimique, telles que définies précédemment dans la demande. Les résultats pour le présent dispositif sont les suivants :

| **Température stabilité : 54°C** | **J0** | **J14** |
|---|---|---|
| **Stabilité physique** | Pas d'exsudation, bonne tenue mécanique | Pas d'exsudation, bonne tenue mécanique |
| **Stabilité chimique (% de l'actif CBD versus quantité initiale incorporée)** | 100% | 95.5 % |

| **Lumière UV** | **J0** | **J7** |
|---|---|---|
| **Stabilité physique** | Pas d'exsudation, bonne tenue mécanique | Pas d'exsudation, bonne tenue mécanique |

Les résultats montrent que le dispositif est stable et a permis d'incorporer efficacement les actifs souhaités, dont le CBD.

### Exemple 6 : Bracelet apaisant

| **Composition** | **Concentration (% p/p)** |
|---|---|
| EVA | QSP 100 |
| Squalane | 1 |
| adipate de diméthyle, glutarate de diméthyle, et succinate de diméthyle | 8.5 |
| Menthe poivrée | 0.75 |
| Isolat de CBD | 0.75 |
| Colorant | 0.1 |

Introduire le polymère dans un réacteur préalablement chauffé à la température requise en fonction de la nature du polymère, ici pour l'EVA autour de 70°C, à une température suffisante pour conférer une structure au polymère permettant d'ouvrir les réseaux pour y incorporer les actifs. Mettre sous agitation.

Préparer la phase active liquide selon le protocole suivant :
- Peser et mélanger les ingrédients de type plastifiants et vectorisants, et mettre sous agitation pour homogénéiser afin d'obtenir un mélange limpide,
- Sous agitation à température ambiante, ajouter l'isolat de CBD en tant que premier actif et laisser agiter jusqu'à obtention d'un mélange limpide translucide
- Ajouter le co-actif, ici l'huile essentielle de menthe poivrée et homogénéiser pour obtenir un mélange translucide homogène,

Une fois que le polymère est à la température cible, ajouter progressivement le mélange d'actif préparé précédemment.

Laisser agiter jusqu'à ce que la phase active soit totalement incorporée dans le.

Refroidir à Température ambiante en maintenant l'agitation.

Ajouter le colorant et homogénéiser le mélange.

Vidanger le réacteur et procédé aux étapes d'injection-moulage afin d'obtenir la forme souhaitée du dispositif

### Exemple 7 : Bracelet anti-douleur

| **Composition** | **Concentration (% p/p)** |
|---|---|
| EVA (*) | QSP 100 |
| Myristate d'isopropyle | 3 |
| Linalol | 2.5 |
| Huile de chanvre riche en CBD | 0.9 |
| Ethylhexyldiphenyl phosphate | 7 |
| Colorant | 0.1 |

Le procédé de fabrication est similaire à celui de l'exemple 6 ci-dessus

### Exemple 8 : Bracelet anti-douleur

| **Composition** | **Concentration (% p/p)** |
|---|---|
| TPU | QSP 100 |
| Myristate d'isopropyle | 3 |
| Linalol | 2.5 |
| Huile de chanvre riche en CBD | 0.9 |
| Ethylhexyldiphenyl phosphate | 17 |
| Colorant | 0.2 |

Le procédé de fabrication est similaire à celui de l'exemple 5 ci-dessus.

### Exemple 9 : Collier apaisant

| **Composition** | **Concentration (% p/p)** |
|---|---|
| EVA (*) | QSP 100 |
| Myristate d'isopropyle | 3.00 |
| Eucalyptus citriodora | 0.75 |
| Menthe poivrée | 0.40 |
| Huile de chanvre riche en CBD | 0.90 |
| Ethylhexyldiphenyl phosphate | 8.50 |
| Tocopherol | 1.5 |
| Colorant | 0.1 |

Le procédé de fabrication est similaire à celui de l'exemple 6 ci-dessus.

### Exemple 10 : Collier apaisant

| **Composition** | **Concentration (% p/p)** |
|---|---|
| TPU | QSP 100 |
| Myristate d'isopropyle | 3.00 |
| Eucalyptus citriodora | 0.75 |
| Huile essentielle de Menthe poivrée | 0.75 |
| Huile de chanvre riche en CBD | 0.90 |
| Ethylhexyldiphenyl phosphate | 8.50 |
| Adipate de diméthyle, glutarate de diméthyle, et succinate de diméthyle | 10 |
| Colorant | 0.5 |

Le procédé de fabrication est similaire à celui de l'exemple 5 ci-dessus.

### Exemple 11 : Collier apaisant

| **Composition** | **Concentration (% p/p)** |
|---|---|
| Squalane | 1 |
| Isolat de CBD | 0.75 |
| Huile essentielle de menthe poivrée | 0.75 |
| Ethylhexyldiphenyl phosphate | 15 |
| adipate de diméthyle, glutarate de diméthyle, et succinate de diméthyle | 13.5 |
| Colorant | 0.1 |
| TPU | QSP 100 |

Le procédé de fabrication est similaire à celui de l'exemple 5 ci-dessus

Les résultats de stabilité pour le présent dispositif sont les suivants :

| **Température stabilité : 54°C** | **J0** | **J14** |
|---|---|---|
| **Stabilité physique** | Pas d'exsudation, bonne tenue mécanique | Pas d'exsudation, bonne tenue mécanique |
| **Stabilité chimique (% de l'actif CBD versus quantité initiale incorporée)** | 100% | 97% |

| **Lumière UV** | **J0** | **J7** |
|---|---|---|
| **Stabilité physique** | Pas d'exsudation, bonne tenue mécanique | Pas d'exsudation, bonne tenue mécanique |

Les résultats montrent que le dispositif est stable et a permis d'incorporer efficacement les actifs souhaités, dont le CBD.

### Exemple 12 : Etude in vitro de la cinétique de libération du CBD à partir des dispositifs selon l'invention

Un test *in vitro* a été mis au point par la demanderesse afin de pouvoir vérifier que l'actif incorporé de façon stable dans le dispositif selon l'invention pouvait aussi être effectivement relargué sur une période de temps allant jusqu'à au moins 4 semaines, afin de traiter le sujet sur lequel le dispositif est appliqué.

Le choix d'une cinétique dans l'huile simule le contact du dispositif avec le sébum de la peau ou du pelage qui permet la libération des actifs pour traiter le sujet.

Des exemples de colliers pour chien précédemment réalisés selon les exemples décrit ci-dessus ont été testés. Certains tests sont toujours en cours à la date de dépôt de la présente demande.

Le mode opératoire de cette analyse pour les différents exemples de colliers est le suivant :
1) Oter la nappe d'enroulement maintenant le collier enroulé, avant utilisation,
2) Peser et noter le poids initial du collier,
3) Introduire le collier dans un flacon de 250 ml,
4) Ajouter de l'huile d'olive jusqu'à totale immersion du collier, et peser,
5) Mettre sous agitation et maintenir l'agitation pendant le temps complet de l'étude cinétique,
6) A chaque temps déterminé de l'analyse (J1, J3, J7, J10, J14, J21, J28 par exemple), réaliser un prélèvement d'huile,
7) Extraire le CBD de l'huile de ce prélèvement à l'aide d'un solvant approprié,
8) Analyser l'extrait en HPLC pour quantifier la quantité de CBD relarguée dans le milieu. On obtient une quantité en mg ou un pourcentage par rapport à la quantité initialement incorporée. L'analyse est réalisée par HPLC phase inverse, avec étalonnage externe à partir d'un standard CBD certifié.
9) Tracer la courbe de libération du CBD dans l'huile en fonction du temps.

Les résultats sont obtenus en moyennant les valeurs de 3 dispositifs similaires testés.

### A) Résultats de la libération du CBD à partir d'un collier selon l'exemple 10 :

| | Qté dosée CBD dans colliers en mg (moyenne de 3 colliers) |
|---|---|
| Jour | % libération |
| 0 | 0% |
| 1 | 13% |
| 3 | 21% |
| 7 | 29% |
| 9 | 31% |
| 10 | 32% |
| 14 | 38% |
| 15 | 39% |
| 21 | 44% |
| 28 | 47% |

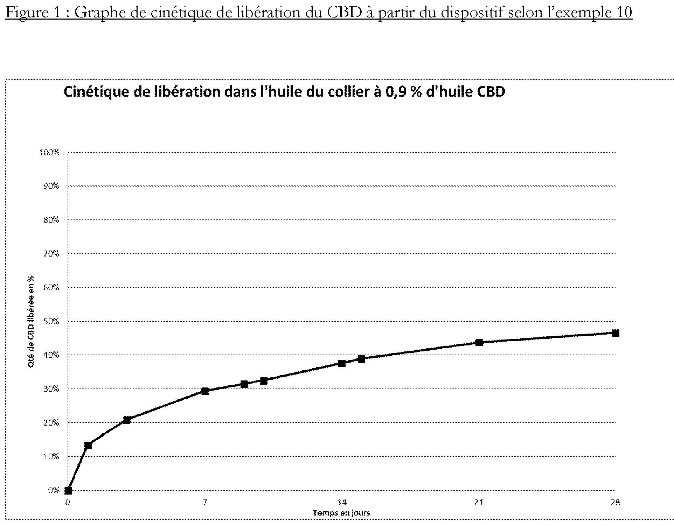

Le dispositif selon l'invention selon l'exemple 10 sous forme de collier a permis d'incorporer efficacement et de façon stable les actifs souhaités, dont le CBD. Les résultats ci-dessus et la courbe correspondante de la Figure 1 prouvent une libération progressive avec le temps du CBD pour un traitement longue durée du sujet à traiter, telle chien. A 28 jours, il peut être noter que le plateau n'est pas atteint et que la libération se poursuit.

### B) Résultats de libération du CBD à partir d'un collier selon l'exemple 5.

| | Qté dosée CBD dans colliers en mg (moyenne de 3 colliers) |
|---|---|
| Jour | %libération |
| 0 | 0% |
| 1 | 14% |
| 3 | 23% |
| 7 | 31% |
| 10 | 34% |
| 14 | En cours |
| 15 | En cours |
| 21 | En cours |
| 28 | En cours |

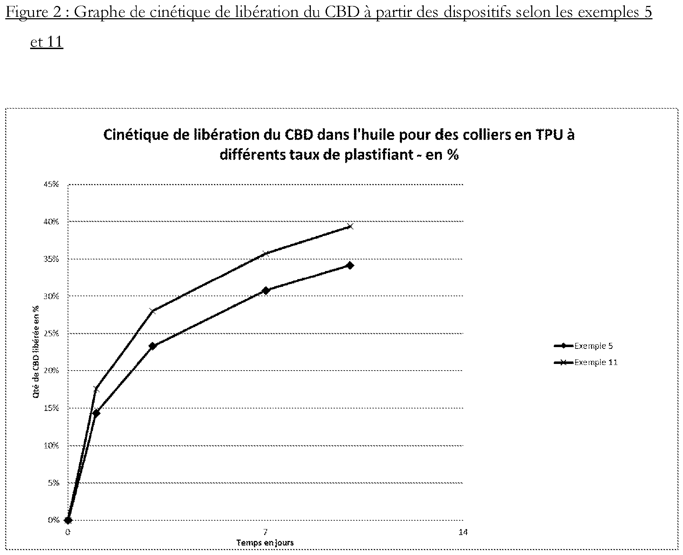

Le dispositif selon l'invention selon l'exemple 5 sous forme de collier a permis d'incorporer efficacement les actifs souhaités, dont le CBD. Les résultats ci-dessus et la courbe correspondante de la Figure 2 prouvent une libération progressive avec le temps du CBD. Les tests en cours permettent d'apprécier la libération sur 10 jours. Il est entendu que le test se poursuit et que l'inclinaison de la courbe permet d'extrapoler aisément quant à la poursuite de la libération avec le temps

### C) Résultats de la libération du CBD à partir d'un collier selon l'exemple 11.

| | Qté dosée CBD dans colliers en mg (moyenne de 3 colliers) |
|---|---|
| Jour | %libération |
| 0 | 0% |
| 1 | 18% |
| 3 | 28% |
| 7 | 36% |
| 10 | 39% |
| 14 | En cours |
| 15 | En cours |
| 21 | En cours |
| 28 | En cours |

Le dispositif selon l'invention selon l'exemple 11 sous forme de collier a permis d'incorporer efficacement les actifs souhaités, dont le CBD. Les résultats ci-dessus et la courbe correspondante de la Figure 2 prouvent une libération progressive avec le temps du CBD. Les tests en cours permettent d'apprécier la libération sur 10 jours. Il est entendu que le test se poursuit et que l'inclinaison de la courbe permet d'extrapoler aisément quant à la poursuite de la libération avec le temps.

La courbe de la Figure 2 permet également de comparer les cinétiques de libération des dispositifs selon l'exemple 5 et 11. Ces deux dispositifs selon l'invention diffèrent uniquement par la quantité de plastifiant au sein de la composition.

Ces résultats démontrent que le taux de composés plastifiants au sein de la composition permet de moduler la cinétique de libération des actifs des dispositifs selon l'invention.

## Revendications

1. Dispositif d'application topique constitué d'une matrice monocouche en polymère **caractérisé en ce que** le polymère est choisi parmi le groupe consistant en un copolymère d'éthylène et d'acétate de vinyle ou un polyuréthane, ladite matrice étant également **caractérisée en ce qu'**elle comprend au moins du cannabidiol (CBD) exempt de delta-9-tétrahydrocannabinol (THC) et entre 0.5% et 20% en poids total de la matrice d'au moins une huile essentielle ou un dérivé monoterpénique des huiles essentielles.

2. Dispositif selon la revendication 1, **caractérisé en ce que** le polymère est non-adhésif.

3. Dispositif selon la revendication 1 ou 2, **caractérisé en ce que** le polymère est un copolymère d'éthylène et d'acétate de vinyle.

4. Dispositif selon la revendication 1 ou 2, **caractérisé en ce que** le polymère et un polyuréthane.

5. Dispositif selon l'une des revendications 1 à 4, **caractérisé en ce que** les huiles essentielles ou dérivés monoterpéniques des huiles essentielles sont choisis parmi le linalol, le menthol ou les huiles essentielles de lavande, lavandin, helicryse italienne, cèdre, citron, citronnelle, carotte, gingembre, niaouli, orange douce, clou de girofle, Eucalyptus citriodora, Eucalyptus radiata, menthe poivrée, géranium, valériane, Nepeta Cataria, camomille, Ylan Ylang, sauge sclarée, mandarine, bois de santal, bergamote, marjolaine, encens, géranium, thym, genévrier ou leurs mélanges.

6. Dispositif selon l'une des revendications 1 à 5 **caractérisé en ce qu'**il comprend un composé vectorisant choisi parmi les huiles d'origine organique, les huiles d'origine synthétique, les huiles d'origine minérale, les huiles d'origine végétale, les esters tels que les mélanges de succinate, glutarate et adipate, les huiles de type acide gras polyinsaturé oméga 3, les esters d'acide gras, les éthers de glycol, le squalène et les dérivés de squalène.

7. Dispositif selon l'une des revendications 1 à 6 **caractérisé en ce qu'**il comprend un composé plastifiant choisi parmi le phosphate d'ethylhexyldiphenyl, l'adipate de dioctyle, l'adipate de diisooctyle, l'adipate de dibutyle, l'adipate de diméthyle, le glutarate de diméthyle, le succinate de diméthyle, ou un mélange de ceux-ci.

8. Dispositif selon l'une des revendications 1 à 7, **caractérisé en ce qu'**il se présente sous la forme d'un collier, d'un bracelet, d'un médaillon ou d'une enduction textile.

9. Dispositif selon l'une des revendications 1 à 8 **caractérisé en ce que** le cannabidiol est présent sous forme d'un isolat de CBD ou d'une huile de CBD.

10. Dispositif selon l'une des revendications 1 à 9 pour son utilisation dans le soulagement du stress ou des douleurs musculaires, articulaires, ou rhumatismales, telles les tendinites, arthrite et l'arthrose.

11. Procédé de fabrication d'un dispositif consistant en une matrice polymère thermoplastique comprenant au moins un polymère thermoplastique non-adhésif choisi parmi un polyuréthane ou un copolymère d'éthylène et d'acétate de vinyle, ladite matrice étant également **caractérisée en ce qu'**elle comprend au moins un isolat de cannabidiol, exempt de delta-9-tétrahydrocannabinol ou une huile de cannabidiol exempte de de delta-9-tétrahydrocannabinol et entre 0.5 et 20% p/p d'au moins une huile essentielle ou un composé d'une huile essentielle, ledit procédé comprenant les étapes de mise en température choisie du polymère, de préparation de la phase active, de mélange de la phase active avec le polymère, puis la mise en forme du dispositif, **caractérisé en ce que** ledit procédé ne comporte pas d'étape d'addition d'excipients spécifiques ou de structure particulaires pour aider à l'incorporation des actifs au sein de la matrice polymère.

## Patentansprüche

1. Vorrichtung zur topischen Anwendung, die aus einer einlagigen Polymermatrix gebildet ist, **dadurch gekennzeichnet, dass** das Polymer ausgewählt ist aus der Gruppe bestehend aus einem Ethylen-Vinylacetat-Copolymer und einem Polyurethan, wobei die Matrix auch **dadurch gekennzeichnet ist, dass** sie mindestens Cannabidiol (CBD), das frei von Delta-9-Tetrahydrocannabinol (THC) ist, und zwischen 0,5 % und 20 %, bezogen auf das Gesamtgewicht der Matrix, ein essentielles Öl oder ein Monoterpenderivat von essentiellen Ölen umfasst.

2. Vorrichtung nach Anspruch 1, **dadurch gekennzeichnet, dass** das Polymer nicht klebend ist.

3. Vorrichtung nach Anspruch 1 oder 2, **dadurch gekennzeichnet, dass** das Polymer ein Ethylen-Vinylacetat-Copolymer ist.

4. Vorrichtung nach Anspruch 1 oder 2, **dadurch gekennzeichnet, dass** das Polymer ein Polyurethan ist.

5. Vorrichtung nach einem der Ansprüche 1 bis 4,
**dadurch gekennzeichnet, dass** die essentiellen Öle oder Monoterpenderivate von essentiellen Ölen ausgewählt sind aus Linalool, Menthol oder essentiellen Ölen von Lavendel, Lavandin, italienischem Helichrysum, Zeder, Zitrone, Citronella, Karotte, Ingwer, Niaouli, Süßorange, Nelke, Eucalyptus citriodora, Eucalyptus radiata, Pfefferminze, Geranie, Baldrian, Nepeta cataria, Kamille, Ylang Ylang, Muskatellersalbei, Mandarine, Sandelholz, Bergamotte, Majoran, Weihrauch, Geranie, Thymian, Wacholder oder Mischungen davon.

6. Vorrichtung nach einem der Ansprüche 1 bis 5,
**dadurch gekennzeichnet, dass** sie eine vektorisierende Verbindung umfasst, die aus Ölen organischen Ursprungs, Ölen synthetischen Ursprungs, Ölen mineralischen Ursprungs, Ölen pflanzlichen Ursprungs, Estern wie Succinat-, Glutarat- und Adipatmischungen, mehrfach ungesättigten Omega-3-Fettsäureölen, Fettsäureestern, Glykolethern, Squalen und Squalenderivaten ausgewählt ist.

7. Vorrichtung nach einem der Ansprüche 1 bis 6,
**dadurch gekennzeichnet, dass** sie eine weichmachende Verbindung umfasst, die aus Ethylhexyldiphenylphosphat, Dioctyladipat, Diisooctyladipat, Dibutyladipat, Dimethyladipat, Dimethylglutarat, Dimethylsuccinat oder einer Mischung davon ausgewählt ist.

8. Vorrichtung nach einem der Ansprüche 1 bis 7,
**dadurch gekennzeichnet, dass** sie in Form eines Halsbands, Armbands, eines Medaillons oder einer Textilbeschichtung vorliegt.

9. Vorrichtung nach einem der Ansprüche 1 bis 8,
**dadurch gekennzeichnet, dass** das Cannabidiol in Form eines CBD-Isolats oder eines CBD-Öls vorliegt.

10. Vorrichtung nach einem der Ansprüche 1 bis 9 zur Verwendung bei der Linderung von Stress oder Muskel-, Gelenk- oder rheumatischen Schmerzen wie Tendinitis, Arthritis und Arthrose.

11. Verfahren zum Herstellen einer Vorrichtung, bestehend aus einer thermoplastischen Polymermatrix, die mindestens ein nicht-haftendes thermoplastisches Polymer umfasst, das aus einem Polyurethan oder einem Ethylen-Vinylacetat-Copolymer ausgewählt ist, wobei die Matrix ferner **dadurch gekennzeichnet ist, dass** sie mindestens ein Cannabidiolisolat, das frei von Delta-9-Tetrahydrocannabinol ist, oder ein Cannabidiolöl, das frei von Delta-9-Tetrahydrocannabinol ist und zwischen 0,5 % und 20 % p/p mindestens eines essentiellen Öls oder einer Verbindung eines essentiellen Öls umfasst, wobei das Verfahren die Schritte des Erwärmens des Polymers auf die gewählte Temperatur, des Herstellens der aktiven Phase, des Mischens der aktiven Phase mit dem Polymer, dann des Formgebens der Vorrichtung umfasst,
**dadurch gekennzeichnet, dass** das Verfahren keinen Schritt des Zugebens von spezifischen Exzipienten oder Partikelstrukturen umfasst, um das Einbauen der Wirkstoffe in die Polymermatrix zu unterstützen.

## Claims

1. A device for topical application formed of a single-layer polymer matrix **characterized in that** the polymer is chosen from the group consisting of an ethylene vinyl acetate copolymer and a polyurethane, said matrix also being **characterized in that** it comprises at least cannabidiol (CBD) free of delta-9-tetrahydrocannabinol (THC) and between 0.5% and 20% by total weight of the matrix of an essential oil or a monoterpenic derivative of essential oils.

2. The device according to claim 1, **characterized in that** the polymer is non-adhesive.

3. The device according to claim 1 or 2, **characterized in that** the polymer is an ethylene vinyl acetate copolymer.

4. The device according to claim 1 or 2, **characterized in that** the polymer is a polyurethane.

5. The device according to any one of claims 1 to 4, **characterized in that** the essential oils or monoterpenic derivatives of essential oils are chosen from linalool, menthol or essential oils of lavender, lavandin, Italian helichrysum, cedar, lemon, citronella, carrot, ginger, niaouli, sweet orange, clove, Eucalyptus citriodora, Eucalyptus radiata , peppermint, geranium, valerian, Nepeta cataria , chamomile, ylang ylang, clary sage, mandarin, sandalwood, bergamot, marjoram, frankincense, geranium, thyme, juniper or mixtures thereof.

6. The device according to any one of claims 1 to 5, **characterized in that** it comprises a vectorizing compound chosen from oils of organic origin, oils of synthetic origin, oils of mineral origin, oils of plant origin, esters such as succinate, glutarate and adipate mixtures, omega 3 polyunsaturated fatty acid oils, fatty acid esters, glycol ethers, squalene and squalene derivatives.

7. The device according to any one of claims 1 to 6, **characterized in that** it comprises a plasticizing compound chosen from ethylhexyl diphenyl phosphate, dioctyl adipate, diisooctyl adipate, dibutyl adipate, dimethyl adipate, dimethyl glutarate, dimethyl succinate, or a mixture thereof.

8. The device according to any one of claims 1 to 7, **characterized in that** it is presented in the form of a collar, bracelet, medallion or textile coating.

9. The device according to any one of claims 1 to 8, **characterized in that** the cannabidiol is present in the form of a CBD isolate or a CBD oil.

10. The device according to any one of claims 1 to 9 for use in the relief of stress or muscular, joint or rheumatic pain, such as tendinitis, arthritis and arthrosis.

11. A method of manufacturing a device consisting of a thermoplastic polymer matrix comprising at least one non-adhesive thermoplastic polymer chosen from a polyurethane or an ethylene vinyl acetate copolymer, said matrix being also **characterized in that** it comprises at least one cannabidiol isolate, free of delta-9-tetrahydrocannabinol or a cannabidiol oil free of delta-9-tetrahydrocannabinol and between 0.5% and 20% p/p of at least one essential oil or a compound of an essential oil, said method comprising the steps of heating the polymer to the chosen temperature, preparing the active phase, mixing the active phase with the polymer, then shaping the device, **characterized in that** said method does not comprise a step of adding specific excipients or particulate structures to help incorporate the active ingredients in the polymer matrix.
